# EUROPEAN PATENT APPLICATION

(11) **EP 4 505 964 A1**
(43) Date of publication of application: **12.02.2025**
(21) Application number: 23806659.1
(22) Date of filing: 14.04.2023
(51) Int. Cl.: A61B 34/30

(54) **MEDICAL INSTRUMENT, SURGICAL ROBOT, AND CONTROL SYSTEM FOR SURGICAL ROBOT**

(30) Priority: 16.05.2022 CN 202210531435
(71) Applicant: Cornerstone Technology (Shenzhen) Limited, Shenzhen Guandong 518066 (CN)
(72) Inventor: CHEN, Zihan, Shenzhen, Guangdong 518000 (CN); YANG, Qiusheng, Shenzhen, Guangdong 518000 (CN); WANG, Zerui, Shenzhen, Guangdong 518000 (CN)
(74) Representative: Balder IP Law, S.L.
(86) International application number: PCT/CN2023/088523
(87) International publication number: WO 2023/221706

(57) **Abstract**

Provided are a medical instrument, a surgical robot and a control system for the surgical robot. The medical instrument is used for the surgical robot and includes a housing and an activation device. The activation device is disposed in the housing and can be triggered. In response to the activation device being operated, the activation device is triggered to generate an induction signal. The activation device transmits the induction signal to an energy generator to enable the energy generator to generate energy and be activated.

## Description

### CROSS REFERENCE TO RELATED APPLICATIONS

This application claims priority to Chinese Patent Application No. 202210531435.3, titled "MEDICAL INSTRUMENT, SURGICAL ROBOT AND CONTROL SYSTEM FOR A SURGICAL ROBOT", filed on May 16, 2022, which is incorporated herein by reference in its entirety.

### TECHNICAL FIELD

The present disclosure generally relates to the technical field of surgical robots, and more specifically to a medical instrument for a surgical robot, a surgical robot, and a control system for a surgical robot.

### BACKGROUND

For safety reasons, the existing surgical instrument such as ultrasonic scalpel need to be connected to an ultrasonic scalpel transducer and an energy generator before use, and a self-test operation can be performed only by connecting the handheld ultrasonic scalpel instrument to an activation device externally. The instrument can be activated only in the case of passing the self-test, and thus used in surgery.

For an existing minimally invasive robotic surgical instrument, an ultrasonic scalpel can be subjected to activation and self-test operations only through an activation device connected to an energy generator before use by a user. In self-test, the energy generator is disconnected from a console in the minimally invasive surgical robotic system first, and then is connected to the activation device. After the self-test is completed, the activation device is removed, and the energy generator is reconnected to the console, enabling a surgeon at the console side to control the energy generator and the ultrasonic instrument.

In addition, during the use of the surgical robotic ultrasonic scalpel, users need to activate the instrument before performing a cleaning operation. In an existing minimally invasive surgical robotic system, this requires disconnection of the energy generator from the console and connection of the energy generator to the activation device again. After the cleaning operation is completed, the activation device is removed and the energy generator is reconnected to the console. This design is, on the one hand, to ensure that only after the energy generator is activated can the ultrasonic instrument be used and controlled by the console, avoiding the situation where the activation device and the robotic system simultaneously control the energy generator.

However, in the above-mentioned usage scenarios, switching between the connection of the activation device to the energy generator and the connection of the robotic system control device to the energy generator interrupts the operation flow of a first user, which affects the user experience. Secondly, alternatingly connecting the console and the activation device to the energy generator is required, which is usually achieved through cables, plugs, and interfaces to ensure the effectiveness of the connection. Frequent plugging and unplugging operations can cause loosening of the connection of the plug at the interface, affecting the safety of the connection. This can reduce the service life of the surgical robotic equipment, and pose a threat to the safety of a patient.

### SUMMARY

A series of simplified concepts are introduced in the Summary, which will be further described in the detailed descriptions. The Summary of the present disclosure does not intend to limit the key and essential technical features of the technical solutions to be protected, nor does it intend to determine the protection scope of the technical solutions as claimed.

In a first aspect of the present disclosure, a medical instrument for a surgical robot is provided. The surgical robot includes a robotic arm, a transducer, and an energy generator. The medical instrument is configured to be mounted on the robotic arm and to be connected to the transducer that is electrically connected to the energy generator. The medical instrument includes a housing and an activation device disposed in the housing. The activation device is configured to be triggered to generate an induction signal and to transmit the induction signal to the energy generator, so as to enable the energy generator to generate energy.

In the present disclosure, the medical instrument of the surgical robot includes an activation device, and an induction signal can be transmitted to the energy generator by operating the activation device on the medical instrument, thereby causing the energy generator to generate energy and be activated.

In some embodiments, the activation device is configured as a physical trigger device including an induction signal generator and an operating member. The induction signal generator is disposed in the housing, and the operating member is disposed on the housing and configured not to protrude from an outer surface of the housing. The operating member is further configured to trigger, in response to being operated, the induction signal generator to generate the induction signal, and the induction signal generator is configured to transmit the induction signal to the energy generator.

In the present disclosure, when the medical instrument is installed on the surgical robot, the operating member is not exposed, avoiding the user from accidentally touching the operating member and ensuring the safety during the use of the medical instrument.

In some embodiments, the housing is configured to be partially recessed inwardly to form an operating area, and the operating member is disposed in the operating area of the housing.

In some embodiments, the operating member is located on a side of the housing facing the robotic arm.

In the present disclosure, when the medical instrument is installed on the robotic arm, the back side of the housing faces the robotic arm, and the operating member is hidden on the back side of the housing to avoid accidental touch by the user.

In some embodiments, the operating member is movably disposed on the housing between a first position where the induction signal generator is not triggered and a second position where the induction signal generator is triggered.

In some embodiments, the operating member includes an elastic connecting portion and a protruding portion, and is connected to the housing through the elastic connecting portion. In response to the operating member being operated, the elastic connecting portion is deformed to move the protruding portion from the first position to the second position.

In some embodiments, the induction signal generator is a push-button switch, and the operating member is an operating button embedded in the housing.

In some embodiments, an operating surface of the operating button is configured to be flush with the outer surface of the housing.

In some embodiments, an operating surface of the operating button is configured to be at least partially recessed into the housing.

In the present disclosure, when the medical instrument is installed on the robotic arm, the operation button does not collide or interfere with other members of the surgical robot, ensuring safety.

In some embodiments, the activation device is configured to be electrically connected to the energy generator through a wired or wireless connection.

In some embodiments, the activation device is electrically connected to the transducer to transmit the induction signal to the energy generator through the transducer.

In some embodiments, the housing is further internally provided with a pin connection device including a pin and a contact. One of the pin and the contact is disposed within the housing and electrically connected to the activation device, and the other one of the pin and the contact is disposed on the transducer. The housing is provided with a transducer interface, and the pin is electrically connected to the contact in response to the transducer being installed in the transducer interface.

In some embodiments, the medical instrument further includes an ultrasonic scalpel assembly or a vessel sealer.

In a second aspect of the present disclosure, a surgical robot is provided. The surgical robot includes a robotic arm, a transducer, an energy generator, and the preceding medical instrument. The medical instrument is operably mounted on the robotic arm, the transducer is electrically connected to the energy generator, the transducer is operably connected to the medical instrument, and the energy generator is configured to receive the induction signal and generate energy based on the induction signal.

The surgical robot in the embodiments of the present disclosure includes the energy generator, the transducer, the robotic arm, and the preceding medical instrument. When the energy generator needs to be activated during use, the surgeon on the patient side can directly activate the energy generator through the medical instrument to generate energy, without frequently switching the connection status between the energy generator and the remote console, making the operation process smoother and more reasonable. This eliminates the need for frequent unplugging and plugging of the plug and interface between the energy generator and the remote console, making the connection between the energy generator and the remote console safer, and thus increasing the service life of equipment of the entire surgical robot.

In a third aspect of the present disclosure, a control system for a surgical robot is provided. The control system includes a medical instrument, an activation device, a transducer, and an energy generator. The activation device is mounted on or not mounted on the medical instrument, the activation device is configured to be triggered to generate an induction signal, and in response to the activation device being operated, a main control unit of the energy generator performs operations described below.

An induction signal is acquired and it is determined whether a self-test operation of the medical instrument has been completed. A vibration frequency signal is triggered in response to the self-test operation having been completed. A self-test is started in response to the self-test operation having not been completed.

The surgical robot control system of the embodiments of the present disclosure includes an activation device. An induction signal is generated by triggering the activation device to activate the energy generator. After receiving the induction signal, the main control unit first performs a self-test for calibration, the actual significance of which is to ensure that the medical instrument is put into use only after the self-test is completed.

In some embodiments, performing the self-test by the main control unit of the energy generator includes at least one of: acquiring information of connectivity between the medical instrument and the transducer; acquiring status information of the medical instrument; and acquiring pre-stored information of the medical instrument.

In some embodiments, the status information of the medical instrument includes physical status information of an end effector of an instrument.

In some embodiments, the pre-stored information of the medical instrument includes identity information of the medical instrument and a number of times the medical instrument has been used.

In some embodiments, the activation device is configured as a physical trigger device, a voice control device, or a gesture control device.

### BRIEF DESCRIPTION OF THE DRAWINGS

The following drawings of the embodiments of the present disclosure are included herein as part of the present disclosure for the purpose of understanding the present disclosure. The drawings illustrate the embodiments of the present disclosure and their descriptions, which are used to explain the principles of the present disclosure. The following drawings are provided.
FIG. 1 is a structure diagram of a surgical robot according to an optional embodiment of the present disclosure.
FIG. 2 is a structure diagram of a surgical robot according to another optional embodiment of the present disclosure.
FIG. 3 is a partial structure view of a surgical robot according to an optional embodiment of the present disclosure.
FIG. 4 is a structure view of a medical instrument according to an optional embodiment of the present disclosure.
FIG. 5 is a perspective view of the medical instrument in FIG. 4.
FIG. 6 is a perspective view illustrating an internal structure of a medical instrument according to an optional embodiment of the present disclosure.
FIG. 7 is a partial structure view of a medical instrument according to another optional embodiment of the present disclosure.
FIG. 8 is a partial structure view of a medical instrument according to yet another optional embodiment of the present disclosure.

### DETAILED DESCRIPTION OF THE EMBODIMENTS

In the following description, many specific details are given to provide a more thorough understanding of the present disclosure. However, it would be apparent to those skilled in the art that the present disclosure may be implemented without one or more of these details. In other examples, some technical features that are well known in the art are not described to avoid confusion with the present disclosure.

For a thorough understanding of the present disclosure, the following provides a detailed description. It should be understood that these embodiments are provided to make the disclosure of the invention thorough and complete, and to adequately convey the idea of these exemplary embodiments to those skilled in the art. Apparently, the embodiments of the present disclosure are not limited in their implementation to particular details familiar to those skilled in the art. Preferred embodiments of the present disclosure are described in detail below. However, the present disclosure may also have other embodiments in addition to these detailed descriptions.

It should be noted that the terms used herein are intended only to describe specific embodiments and are not intended to limit the exemplary embodiments according to the present disclosure. As used herein, the singular form is also intended to include the plural form, unless the context clearly indicates otherwise. Furthermore, it should be further understood that the terms "includes," "including," "comprises," and/or "comprising," when used herein, specify the presence of stated features, integers, steps, operations, elements, and/or components, but do not preclude the presence or addition of one or more other features, integers, steps, operations, elements, components, and/or groups thereof.

Ordinal numbers such as "first" and "second" quoted in the present disclosure are merely identifications, and do not have any other meanings, such as a specific order. Also, for example, the term "first element" does not by itself imply the presence of "second element," nor does the term "second element" by itself imply the presence of "first element."

It should be noted that the terms "upper," "lower," "front," "rear," "left," "right," "inner," "outer" and similar expressions used herein are for the purpose of illustration and not limitation.

Exemplary embodiments of the present disclosure are now described in more detail with reference to the drawings.

The embodiments of the present disclosure provide a medical instrument for a surgical robot and a surgical robot including the medical instrument.

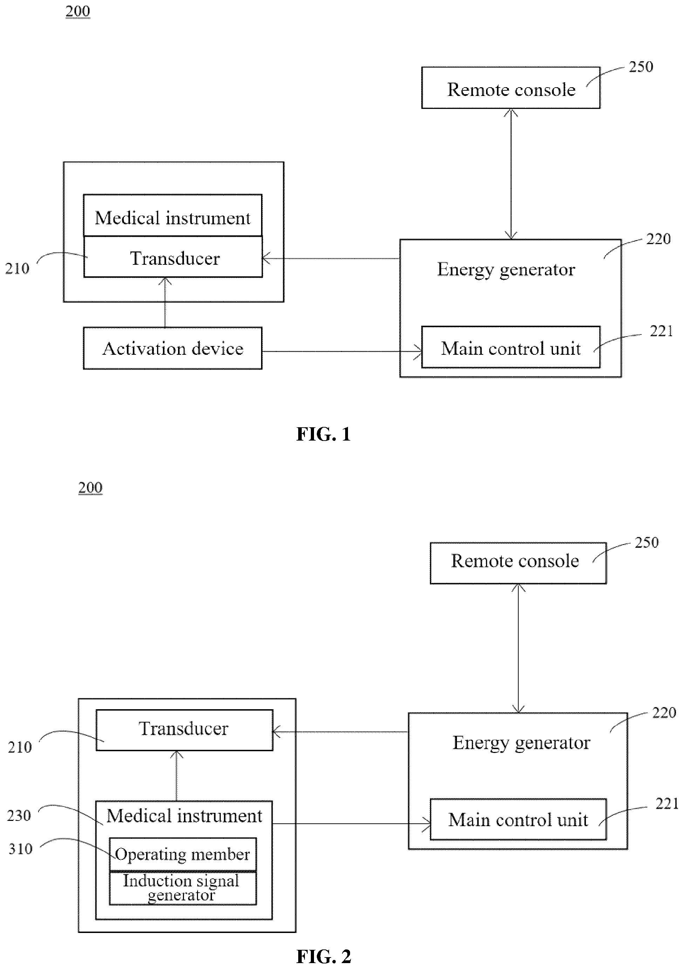

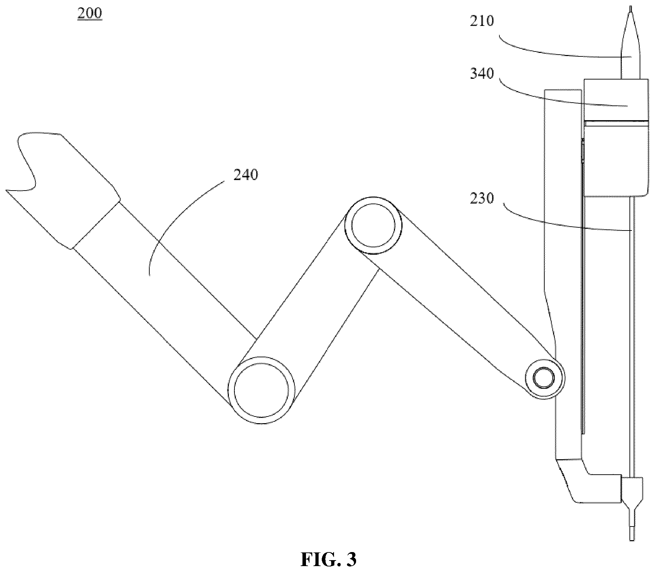

In order to more accurately understand the technical solutions of the present disclosure, the surgical robot is introduced first. As shown in FIGS. 1 and 2, in an embodiment, the surgical robot 200 includes a transducer 210, an energy generator 220, a medical instrument 230, and a robotic arm 240. The medical instrument 230 is configured to be installed on the robotic arm 240, and controlling the change in orientation of the robotic arm 240 can drive the change in orientation of the medical instrument 230. As shown in FIG. 3, the medical instrument 230 is located at the end of the robotic arm 240.

Specifically, the transducer 210 is electrically connected to the energy generator 220 through a cable. The energy generator 220 includes a main control unit 221 and a generator body (not shown). The main control unit 221 can control the generator body to generate energy (such as electric energy) and output the energy to the transducer 210. The transducer 210 is configured to convert the received energy into mechanical energy and transmit the mechanical motion to the medical instrument 230 connected to the transducer 210. The main control unit 221 is communicatively connected to a remote console 250, and the surgeon on the remote console 250 side can input a control command to the main control unit 221, so that the surgeon on the remote console 250 side can control a state of the medical instrument 230 by inputting different control commands to the energy generator 210. The main control unit 221 is specifically connected to the remote console 250 through a cable, which ensures the speed and stability of signal transmission.

It can be understood that during the use of the surgical robot 200 of the present disclosure, the robotic arm 240, the medical instrument 230, the energy generator 220, and the transducer 210 are all disposed next to the patient, while the remote console 250 is disposed at a location remote from the patient.

Based on this connection mode between the main control unit 221 and the remote console 250, as shown in FIGS. 3 to 5, in an optional implementation, the medical instrument 230 according to the present disclosure includes a housing 340 and an activation device disposed in the housing 340.

In some embodiments, the activation device is implemented by a physical trigger device. Referring to FIG. 2, the activation device specifically includes an induction signal generator and an operating member 310.

The induction signal generator is disposed inside the housing 340. The operating member 310 is disposed on the housing 340 and is configured not to protrude from the outer surface of the housing 340. In response to the operating member 310 being operated, the operating member 310 can trigger the induction signal generator to generate an induction signal. The induction signal generator is configured to transmit the induction signal to the energy generator 220 to enable the energy generator 220 to generate energy.

According to a specific implementation of the present disclosure, operating the operating member 310 of the medical instrument 230 can cause the induction signal generator to send an induction signal to the energy generator 220, thereby activating the energy generator 220. By designing the operating member 310 in a concealed manner, the user is prevented from accidentally touching the operating member 310, ensuring the safety during the use of the medical instrument 230. Those skilled in the art can flexibly select and configure the operating member according to the function of the induction signal generator.

Specifically, the induction signal generator is electrically connected to the main control unit 221, and the main control unit 221 activates the energy generating device body 220 based on the induction signal, so that the generating device body can be controlled to generate energy and output the energy to the transducer 210. For example, the energy generator 210 outputs a current or voltage to the transducer 210 based on the induction signal.

Referring to FIG. 3, the operating member 310 is located on a side of the housing 340 facing the robotic arm 240. This prevents a surgeon close to the medical instrument 230 (a surgeon on the patient side) from accidentally touching the operating member 310 during operation, and ensures the safety of the medical instrument 230 during use.

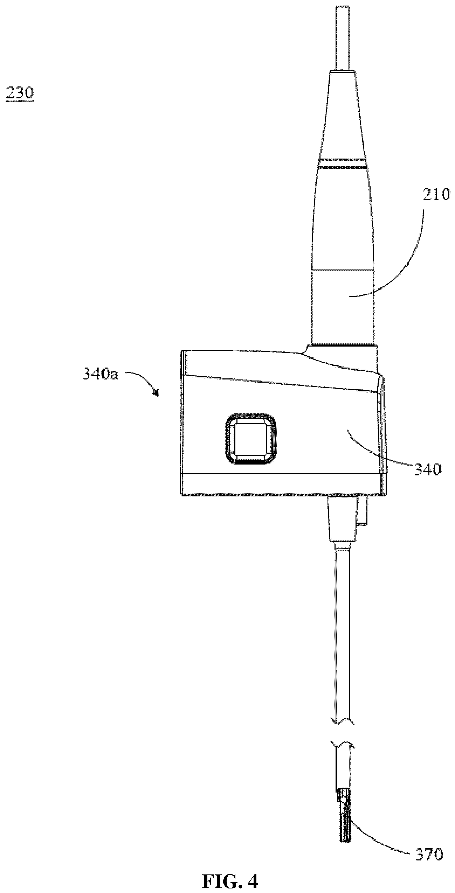

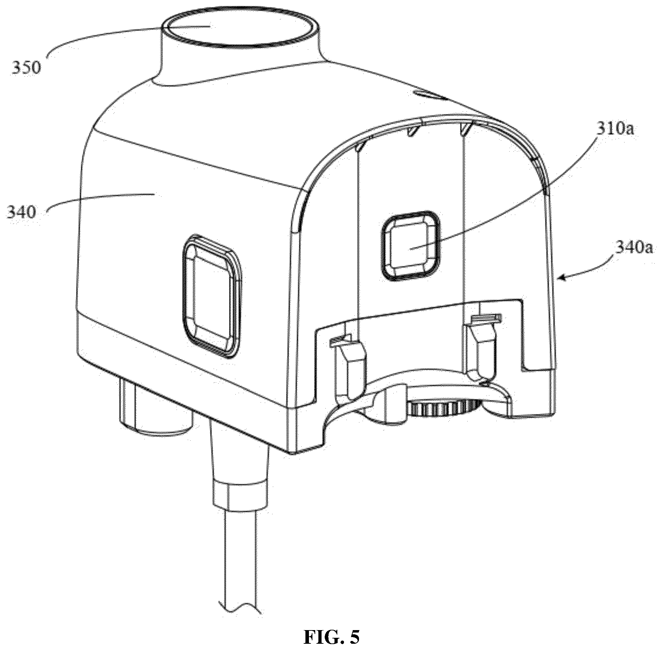

As shown in FIGS. 4 and 5, the housing 340 is configured to be partially recessed inwardly to form an operating area 340a, and the operating member 310 is disposed in the operating area 340a of the housing 340. During the operation of the surgical robot 200, the recessed operating area 340a makes it difficult for the surgeon on the patient side to touch the operating member 310.

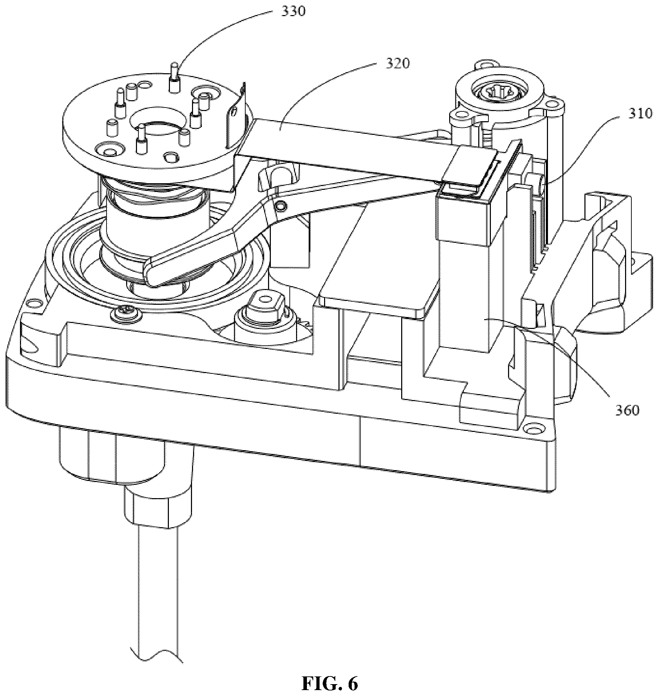

As shown in FIGS. 5 and 6, in the embodiments of the present disclosure, the induction signal generator is implemented by a push-button switch, and the operating member 310 is an operating button. The operating button is embedded in the housing 340, and the push-button switch is disposed in a switch seat 360. In other words, the housing 340 is provided with a through-hole structure for embedding the operating button, and a space for accommodating the operating button is provided inside the housing 340. The push-button switch inside the housing 340 can be triggered by pressing the operating button to generate an induction signal.

Specifically, the operating button has an operating surface 310a, which is configured to be at least partially recessed into the housing 340. As shown in FIG. 5, the operating button is located in the operating area 340a, and an operating surface 310a (the pressed surface) of the operating button is roughly configured as a rounded rectangular surface. The edge of the operating surface 310a is roughly flush with the outer surface of the housing 340 of the operating area 340a, and the center portion of the operating surface 310a is recessed toward the interior of the housing 340.

In the embodiment shown in FIG. 6, the operating surface 310a of the operating button is roughly configured as a circular surface, and the operating button is of a sunken design: the operating surface 310a of the operating button is lower than the outer surface of the housing 340. In this way, when the medical instrument 230 is installed on the robotic arm 240, the operating button 310 does not collide or interfere with other components of the surgical robot 100, further ensuring safety.

Alternatively, in other embodiments of the present disclosure, the operating surface 310a of the operating button can be configured to be flush with the outer surface of the housing 340. The operating buttons described above are all designed in such a way as to achieve the effect of hiding the operating button, further avoiding the surgeon on the patient side from accidentally touching the operating button.

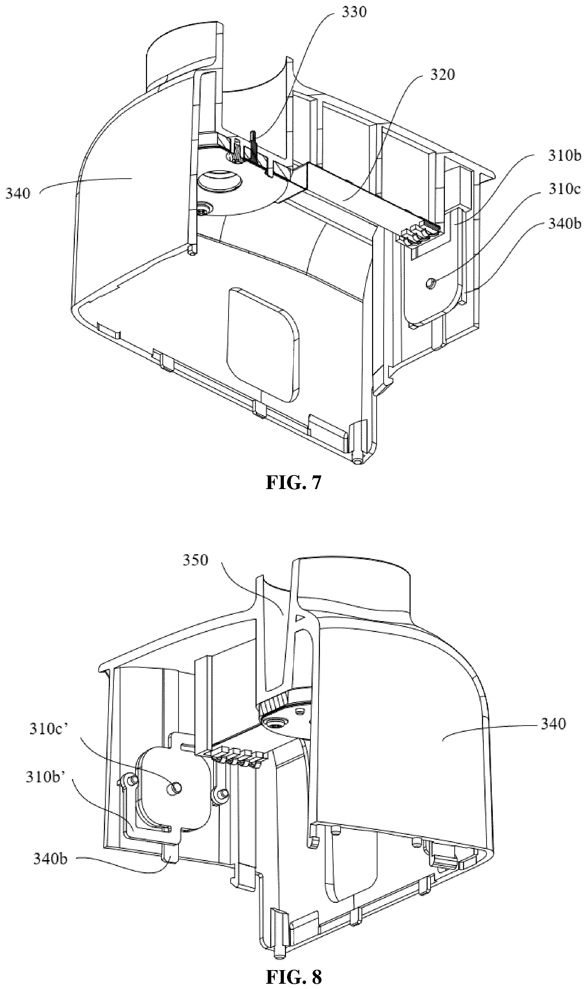

In an optional embodiment of the present disclosure, the operating member 310 is movably disposed in the housing 340. Specifically, the operating member 310 can be moved between a first position where the induction signal generator is not triggered and a second position where the induction signal generator is triggered. Referring to FIGS. 7 and 8, the operating member 310 further includes an elastic connecting portion and a protruding portion, and the operating member 310 is connected to the housing 340 through the elastic connecting portion. In response to the operating member 310 being operated, the elastic connecting portion can be deformed to move the protruding portion of the operating member 310 from the first position to the second position. It can be understood that the elastic connecting portion is made of an elastic material.

In the embodiment shown in FIG. 7, the operating member 310 is an operating button, and the operating button is connected to the housing 340 through an elastic connecting portion 310b disposed on the top or bottom side of the operating member 310. In response to the operating surface 310a of the operating button being pressed, the elastic connecting portion 310b is deformed such that the operating button as a whole is slightly tilted toward the interior of the housing 340 relative to the connection position between the elastic connecting portion 310b and the housing 340 to cause the protruding portion 310c to contact the push-button switch, which triggers the push-button switch to generate an induction signal. In response to the operating surface 310a being released, the operating button as a whole is reset (i.e., being rotated toward the exterior of the housing 340 relative to the connection position between the elastic connecting portion 310b and the housing 340 until the operating button returns to its original position).

In the embodiment shown in FIG. 8, the operating member 310 is an operating button, and the operating button is fixed to the housing 340 through an elastic connecting portion 310b'. The elastic connecting portion 310b' includes two centrally symmetrical L-shaped structures made of an elastic material. In response to the operating surface 310a of the operating button being pressed from outside the housing 340, the two centrally symmetrical L-shaped structures of the elastic connecting portion 310b' in the housing 340 are simultaneously deformed, so that the protruding portion 310c' is biased toward the interior of the housing 340 to contact the push-button switch, thereby triggering the push-button switch to generate an induction signal. In response to the operating surface 310a being released, the operating button is reset under the elastic force generated by the deformation of the elastic connecting portion 310b', disengaging the protruding portion 310c' from the push-button switch. Compared to the embodiment shown in FIG. 7, the centrally symmetrical provision of the two L-shaped structures as the elastic connecting portion 310b' enables more stable and reliable movement of the operating button.

The housing 340 is configured internally with a support portion 340b for supporting the operating member 310. Referring to FIGS. 7 and 8, the housing 340 partially protrudes inward to form the support portion 340b.

Optionally, the induction signal generator is configured to be electrically connected to the energy generator 220 via a wired or wireless connection. Returning to FIG. 2, the arrows in FIG. 2 indicate the signal transmission mode. In one possible implementation, the induction signal generator in the medical instrument 230 can directly transmit an induction signal to the main control unit 221 of the energy generator 220. In FIG. 2, the induction signal generator may also be electrically connected to the transducer 210 and then transmit the induction signal to the main control unit 221 through the transducer 210.

In some other embodiments, the activation device may not use the aforementioned operating button, but instead, a switch button of the push-button switch is directly mount on the housing and designed as a sunken structure to prevent accidental touches.

In the embodiments of the present disclosure, the induction signal generator is electrically connected to the transducer 210 electrically connected to the energy generator 220 through a cable. Thus, the induction signal generator can transmit an induction signal to the energy generator 220 through the transducer 210.

Specifically, the housing is further internally provided with a pin connection device. The pin connection device includes a pin and a contact. One of the pin and the contact is disposed inside the housing and electrically connected to the induction signal generator, and the other one of the pin and the contact is configured to be disposed on the transducer. The housing is provided with a transducer interface for connecting the transducer. In response to the transducer being installed in the transducer interface, the pin is electrically connected to the contact. In other words, when the contact is disposed inside the housing, the pin is disposed on the transducer. Conversely, when the pin is disposed inside the housing, the contact is disposed on the transducer. The pin connection device serves as a convenient electrical connector here, and those skilled in the art can flexibly set the type of the electrical connector according to actual conditions.

In the embodiment shown in FIG. 6, the push-button switch is electrically connected to the transducer 210 through the pin connection device disposed inside the housing 340. The housing 340 of the medical instrument 230 is provided with a transducer interface 350 for connecting the transducer 210, and a pin 330 is disposed on a mounting seat at the transducer interface 350 inside the housing 340. The push-button switch is electrically connected to the pin 330 through a flat cable 320. Correspondingly, the contact of the pin connection device is disposed on the transducer 210. When the transducer 210 is mounted on the mounting seat of the transducer interface 350, the pin 330 is electrically connected to the contact, and the push-button switch is electrically connected to the transducer 210.

In this embodiment, the medical instrument 230 further includes an ultrasonic scalpel assembly. As shown in FIG. 4, the ultrasonic scalpel assembly includes an ultrasonic scalpel tip 370 at a lower end. The ultrasonic scalpel assembly is connected to the transducer 210. The transducer 210 can convert the energy output by the energy generator 220 into mechanical vibration and transmit the mechanical vibration to the ultrasonic scalpel tip 370, causing the ultrasonic scalpel tip 370 to vibrate. Corresponding treatment is completed by heat generated by the high-frequency vibration of the ultrasonic scalpel tip 370. The surgeon on the remote console 250 side can control and adjust the vibration frequency of the ultrasonic scalpel by inputting different control commands to the main control unit 221.

It can be understood that the medical instrument 230 of the present disclosure may further include other medical equipment requiring energy generation, such as a vascular sealer.

Optionally, the medical instrument 230 is operably mounted on the robotic arm 240, and the transducer 210 is operably connected to the medical instrument 230. This facilitates the updating and maintenance of equipment in the surgical robot 200.

Returning to FIG. 1, in other embodiments of the present disclosure, the activation device may not be installed on the medical instrument. For example, the activation device may be integrated or externally connected to the energy generator 220 by using a voice control device, a gesture control device or a physical trigger device, and random triggering is avoided by an additional protection mechanism.

It can be understood that the activation of the energy generator refers to a state in which the energy generator is started, which is known to those skilled in the art. For example, after disconnection and reconnection of the transducer and the medical instrument or when the medical instrument needs to be cleaned, the energy generator needs to be activated to perform the activation self-test or activation cleaning of the medical instrument. Compared with the existing surgical robots, when the energy generator needs to be activated, the surgical robot of the present disclosure allows the surgeon on the patient side to directly activate the energy generator by the operating member and induction signal generator disposed in the medical instrument to generate energy so as to perform the corresponding target operation.

The embodiments of the present disclosure further provide a control system for a surgical robot, including a medical instrument, an activation device, a transducer 210, and an energy generator 220. The activation device can be triggered to generate an induction signal. According to an embodiment of the present disclosure, after receiving the induction signal, the main control unit 221 of the energy generator 220 performs operations described below.

An induction signal is acquired and it is determined whether a self-test operation of the medical instrument has been completed.

A vibration frequency signal is triggered in response to the self-test operation having been completed.

A self-test is started in response to the self-test operation having not been completed.

Performing the self-test by the main control unit of the energy generator includes at least one of: acquiring information of connectivity between the medical instrument and the transducer; acquiring status information of the medical instrument; and acquiring pre-stored information of the medical instrument.

In an embodiment, the self-test further includes operations described below.

In response to the acquired status information of the medical instrument is that an end effector of an instrument is closed, the vibration frequency signal is not triggered.

In response to the acquired status information of the medical instrument being that the end effector of the instrument is open, the vibration frequency signal is triggered and a function test of the medical instrument is started.

In response to the function test of the medical instrument passing, the medical instrument is available; and in response to the function test of the medical instrument not passing, the medical instrument is not available.

The pre-stored information of the medical instrument includes identity information of the medical instrument and a number of times the medical instrument has been used.

The main control unit controls the generator body to generate energy based on the vibration frequency signal and output the energy to the transducer, enabling the transducer to vibrate at a certain vibration frequency.

In addition, during the operation of the surgical robot 200, if it is necessary to clean the medical instrument 230, the energy generator 220 may also be activated to output energy to the transducer 210 through the above operations to vibrate the medical instrument 230. Specifically, when cleaning the ultrasonic scalpel tip 370 during surgery, the surgeon on the patient side presses the operating button to activate the energy generator 220, which then outputs energy to the transducer 210. For example, the energy generator 210 outputs electrical energy (a current or voltage) to the transducer 210 based on the induction signal, and the transducer 210 converts the electrical energy into mechanical energy to drive the ultrasonic scalpel tip 370 to vibrate. The surgeon on the patient side then places the ultrasonic scalpel in a cleaning device for vibration cleaning.

The above operations of activating the energy generator 220 do not require disconnecting the energy generator 220 from the remote console 250, making the operational flow smoother and more reasonable.

Unless otherwise defined, the technical and scientific terms used herein have the same meanings as commonly understood by those skilled in the technical field of the present disclosure. The terms used herein are for the purpose of describing specific embodiments only and are not intended to limit the present disclosure. Terms such as "dispose" used in this document can indicate that one component is directly attached to another component, or that one component is attached to another component through an intermediate component. The features described in one embodiment herein can be applied individually or in combination with other features to another embodiment, unless the feature is not applicable in that other embodiment or otherwise specified.

The present disclosure has been described through the above embodiments, but it should be understood that these embodiments are for illustrative and explanatory purposes only, and are not intended to limit the present disclosure to the described embodiments. Those skilled in the art can understand that many variations and modifications can be made based on the teachings of the present disclosure, and these variations and modifications fall within the scope of protection of the present disclosure.

## Claims

1. A medical instrument for a surgical robot, wherein the surgical robot includes a robotic arm, a transducer, and an energy generator, the medical instrument is configured to be mounted on the robotic arm and to be connected to the transducer that is electrically connected to the energy generator, and the medical instrument comprises:
a housing; and
an activation device, disposed in the housing and configured to be triggered to generate an induction signal and to transmit the induction signal to the energy generator, the energy generator being configured to generate energy in response to receiving the induction signal.

2. The medical instrument according to claim 1, wherein the activation device is configured as a physical trigger device including:
an induction signal generator, disposed in the housing; and
an operating member, disposed on the housing and configured not to protrude from an outer surface of the housing;
wherein the operating member is configured to trigger, in response to being operated, the induction signal generator to generate the induction signal, and the induction signal generator is configured to transmit the induction signal to the energy generator.

3. The medical instrument according to claim 2, wherein the housing is configured to be partially recessed inwardly to form an operating area, and the operating member is disposed in the operating area of the housing.

4. The medical instrument according to claim 2 or 3, wherein the operating member is located on a side of the housing facing the robotic arm.

5. The medical instrument according to any one of claims 2 to 4, wherein the operating member is movably disposed on the housing between a first position where the induction signal generator is not triggered and a second position where the induction signal generator is triggered.

6. The medical instrument according to claim 5, wherein the operating member includes an elastic connecting portion and a protruding portion, and is connected to the housing through the elastic connecting portion; and
wherein in response to the operating member being operated, the elastic connecting portion is deformed to move the protruding portion from the first position to the second position.

7. The medical instrument according to claim 6, wherein the induction signal generator is a push-button switch, and the operating member is an operating button embedded in the housing.

8. The medical instrument according to claim 7, wherein an operating surface of the operating button is configured to be flush with the outer surface of the housing.

9. The medical instrument according to claim 7, wherein an operating surface of the operating button is configured to be at least partially recessed into the housing.

10. The medical instrument according to any one of claims 1 to 9, wherein the activation device is configured to be electrically connected to the energy generator through a wired or wireless connection.

11. The medical instrument according to any one of claims 1 to 9, wherein the activation device is connected to the transducer to transmit the induction signal to the energy generator through the transducer.

12. The medical instrument according to claim 11, wherein the housing is internally provided with a pin connection device including a pin and a contact;
wherein one of the pin and the contact is disposed within the housing and electrically connected to the activation device, and the other one of the pin and the contact is disposed on the transducer; and
wherein the housing is provided with a transducer interface, and the pin is electrically connected to the contact in response to the transducer being installed in the transducer interface.

13. The medical instrument according to any one of claims 1 to 12, further comprising an ultrasonic scalpel assembly or a vessel sealer.

14. A surgical robot, comprising a robotic arm, a transducer, an energy generator, and the medical instrument according to any one of claims 1 to 13, wherein the medical instrument is operably mounted on the robotic arm, the transducer is electrically connected to the energy generator, the transducer is operably connected to the medical instrument, and the energy generator is configured to receive the induction signal and generate energy based on the induction signal.

15. A control system for a surgical robot, comprising a medical instrument, an activation device, a transducer, and an energy generator, wherein the activation device is mounted on or not mounted on the medical instrument, the activation device is configured to be triggered to generate an induction signal, and in response to the activation device being operated, a main control unit of the energy generator performs operations of:
acquiring an induction signal and determining whether a self-test operation of the medical instrument has been completed;
triggering a vibration frequency signal in response to the self-test operation having been completed; and
starting a self-test in response to the self-test operation having not been completed.

16. The control system for the surgical robot according to claim 15, wherein performing the self-test by the main control unit of the energy generator includes at least one of:
acquiring information of connectivity between the medical instrument and the transducer;
acquiring status information of the medical instrument; and
acquiring pre-stored information of the medical instrument.

17. The control system for the surgical robot according to claim 16, wherein the status information of the medical instrument includes physical status information of an end effector of an instrument.

18. The control system for the surgical robot according to any one of claims 15 to 17, wherein the pre-stored information of the medical instrument includes identity information of the medical instrument and a number of times the medical instrument has been used.

19. The control system for the surgical robot according to any one of claims 15 to 18, wherein the activation device is configured as a physical trigger device, a voice control device, or a gesture control device.
